# EUROPEAN PATENT SPECIFICATION

(11) **EP 3 160 487 B1**
(45) Date of publication and mention of the grant of the patent: **01.05.2024**
(21) Application number: 15751073.6
(22) Date of filing: 25.06.2015
(51) Int. Cl.: A61K 38/10, A61P 31/12, A61P 31/10, A61P 31/04, A61L 12/14, A61K 31/155, A61K 38/40, A61K 38/17, A61K 38/16, A61K 38/12, A61K 9/00, A61K 47/18, A61P 31/00, A61P 43/00

(54) **ANTIMICROBIAL COMPOSITIONS FOR THE VETERINARY FIELD**
ANTIMIKROBIELLE ZUSAMMENSETZUNGEN FÜR DIE VETERINÄRMEDIZIN
COMPOSITIONS ANTIMICROBIENNES POUR LE DOMAINE VÉTÉRINAIRE

(30) Priority: 27.06.2014 IT MI20141176
(43) Date of publication of application: 03.05.2017
(73) Proprietor: I.C.F. S.r.l., 26020 Palazzo Pignano (CR) (IT)
(72) Inventor: CABASSI, Clotilde Silvia, 43121 Parma (PR) (IT); FALANGA, Gennaro, 26040 Gerre De' Caprioli (CR) (IT); ROMANI, Antonello, 43125 Parma (PR) (IT)
(74) Representative: Villa, Livia
(86) International application number: PCT/IB2015/054790
(87) International publication number: WO 2015/198265

(56) References cited:
- WO-A2-2009/037183
- US-A1- 2007 003 538
- A. A. ROMANI ET AL: "In vitro activity of novel in silico -developed antimicrobial peptides against a panel of bacterial pathogens", JOURNAL OF PEPTIDE SCIENCE, vol. 19, no. 9, 26 September 2013 (2013-09-26), pages 554-565, XP055119521, ISSN: 1075-2617, DOI: 10.1002/psc.2532
- GIUSEPPANTONIO MAISETTA ET AL: "Activity of Human ss-Defensin 3 Alone or Combined with Other Antimicrobial Agents against Oral Bacteria", ANTIMICROBIAL AGENTS AND CHEMOTHERAPY, AMERICAN SOCIETY FOR MICROBIOLOGY, vol. 47, no. 10, 1 October 2003 (2003-10-01), pages 3349-3351, XP008130454, ISSN: 0066-4804, DOI: 10.1128/AAC.47.10.3349-3351.2003
- KIM S S ET AL: "SYNERGISTIC INHIBITORY EFFECT OF CATIONIC PEPTIDES AND ANTIMICROBIAL AGENTS ON THE GROWTH OF ORAL STREPTOCOCCI", CARIES RESEARCH, S. KARGER AG, BASEL, CH, vol. 37, 1 January 2003 (2003-01-01), pages 425-430, XP008074671, ISSN: 0008-6568, DOI: 10.1159/000073394
- REICHEL M ET AL: "Skin bacteria after chlorhexidine exposure-is there a difference in response to human [beta]-Defensin-3?", EUROPEAN JOURNAL OF CLINICAL MICROBIOLOGY & INFECTIOUS DISEASES, SPRINGER, BERLIN, DE, vol. 29, no. 6, 27 March 2010 (2010-03-27) , pages 623-632, XP019807529, ISSN: 1435-4373
- KEUN-HYEUNG LEE ET AL: "Antimicrobial activity and conformation of gaegurin-6 amide and its analogs", PEPTIDES, vol. 19, no. 10, 1 January 1998 (1998-01-01), pages 1653-1658, XP055168825, ISSN: 0196-9781, DOI: 10.1016/S0196-9781(98)00119-3
- PUSATERI C R ET AL: "Sensitivity of Candida albicans biofilm cells grown on denture acrylic to antifungal proteins and chlorhexidine", ARCHIVES OF ORAL BIOLOGY, PERGAMON PRESS, OXFORD, GB, vol. 54, no. 6, 1 June 2009 (2009-06-01), pages 588-594, XP026128396, ISSN: 0003-9969, DOI: 10.1016/J.ARCHORALBIO.2009.01.016 [retrieved on 2009-02-27]
- ANDRÉ C. AMARAL ET AL: "Predicting antimicrobial peptides from eukaryotic genomes: In silico strategies to develop antibiotics", PEPTIDES, vol. 37, no. 2, 1 October 2012 (2012-10-01), pages 301-308, XP055217886, ISSN: 0196-9781, DOI: 10.1016/j.peptides.2012.07.021

## Description

### FIELD OF THE INVENTION

The present invention relates to compositions comprising chlorhexidine or a salt thereof, and at least one peptide, for use in the treatment of infections caused by bacteria, in the veterinary field, as defined in more detail in the claims.

### BACKGROUND ART

Chlorhexidine is a cationic polybiguanide (bisbiguanide). It is used mainly in the form of salt, for example dihydrochloride, diacetate and digluconate.

As a biocide, its target is the bacterial cell wall. At low concentrations, chlorhexidine binds to the negatively charged cell wall and disrupts the osmotic balance. At higher concentrations, chlorhexidine attacks the bacterial cytoplasmic membrane and denatures the microbial proteins. Chlorhexidine has both a rapid onset of bactericidal action and a prolonged antimicrobial efficacy through residual effects.

Depending on the formulation and concentration, chlorhexidine is effective as bactericide, virucide and fungicide.

In particular, at low concentrations, chlorhexidine is effective against most gram-positive bacteria. At higher concentrations, chlorhexidine is effective against gram-negative bacteria. At the highest concentrations, chlorhexidine is effective against yeasts.

The virucidal activity is good against 'coated' viruses (such as HIV, cytomegalovirus, influenza, respiratory syncytial virus and herpes virus), but not against 'naked' viruses (such as rotaviruses, adenoviruses and enteroviruses).

Chlorhexidine has no sporicidal activity, therefore it is not effective against *Clostridium difficile* spores and it is not active against mycobacteria.

Thanks to its broad activity spectrum, its acceptable tolerability and a good level of safety, chlorhexidine is one of the most frequently used antiseptic agents. The reduced availability of triclosan products following concerns about the safety and the selection of the antimicrobial resistance has exacerbated the growing exposure to chlorhexidine. In addition, there is a growing emphasis on the control of the methicillin-sensitive S. *aureus* (MSSA), which probably increases the further use of products containing chlorhexidine.

In a recent publication by Homer C. et al. ("Reduced susceptibility to chlorhexidine in staphylococci: is it increasing and does it matter?" J Antimicrob Chemother., 2012 Nov; 67(11): 2547-59), it is noted that different methods have been used for the detection of reduced susceptibility to chlorhexidine, but there is no standardized method and there is no consensus on the definition of 'resistance' to chlorhexidine. In particular in this publication, the evidence of reduced susceptibility to chlorhexidine in staphylococci was examined. The authors conclude that "the clinical use of chlorhexidine will continue to increase and it will be important to pay attention to the possibility that this could lead to the emergence of new clones with reduced susceptibility. The indiscriminate use of chlorhexidine in the absence of efficacy data should be discouraged".

A. A. ROMANI ET AL: "In vitro activity of novel in silico -developed antimicrobial peptides against a panel of bacterial pathogens", JOURNAL OF PEPTIDE SCIENCE,vol. 19, no. 9, 26 September 2013 (2013-09-26), pages 554-565,ISSN: 1075-2617, DOI: 10.1002/psc.2532 disclose the peptides AMP2041 (corresponding to present SEQ.ID 1); AMP72 (corresponding to present SEQ.ID 2); and AMP126 (corresponding to present SEQ.ID 3) as peptides having an antimicrobial, more specifically antibacterial activity, including an activity against Pseudomonas aeruginosa and Staphylococcus aureus.

GIUSEPPANTONIO MAISETTA ET AL: "Activity of Human ss-Defensin 3 Alone or Combined with Other Antimicrobial Agents against Oral Bacteria",ANTIMICROBIAL AGENTS AND CHEMOTHERAPY, AMERICAN SOCIETY FOR MICROBIOLOGY,vol. 47, no. 10, 1 October 2003 (2003-10-01), pages 3349-3351, ISSN: 0066-4804, DOI: 10.1128/AAC.47.10.3349-3351.2003 disclose an antibacterial activity of human beta-defensin 3 (hBD-3) against oral bacterial when used alone or in combination with other antimicrobial agents, including chlorhexidine.

US 2007/003538 A1 discloses compositions for preventing growth and proliferation of biofilm embedded microorganisms. The compositions comprise a cationic polypeptide and a bis-guanide, such as chlorhexidine.

KIM S SET AL: "SYNERGISTIC INHIBITORY EFFECT OF CATIONIC PEPTIDES AND ANTIMICROBIAL AGENTS ON THE GROWTH OF ORAL STREPTOCOCCI",CARIES RESEARCH, S. KARGER AG, BASEL, CH,vol. 37, 1 January 2003 (2003-01-01), pages 425-430,ISSN: 0008-6568, DOI: 10.1159/000073394 disclose a synergistic inhibitory effect of cationic peptides and antimicrobial agents on the growth of oral streptococci. Synergistic effects of a combination of the cationic peptides with chlorhexidine and with xylitol were observed.

The object of the present invention is therefore to be able to benefit from the efficacy of chlorhexidine, while to avoid triggering mechanisms of resistance by the microorganisms concerned.

### SUMMARY OF THE INVENTION

The above object has been achieved by a composition comprising chlorhexidine or a salt thereof, and at least one peptide, as reported in claim 1.

The characteristics and the advantages of the present invention will become apparent from the following detailed description and from the working Examples. The invention is defined by the claims. Any subject-matter outside the scope of the claims is provided for reference or comparison purposes only.

Any reference to a non-patentable method of treatment involving a certain compound or composition is to be interpreted as a reference to said compound or composition for use in said method.

### DETAILED DESCRIPTION OF THE INVENTION

The subject of the invention therefore is a composition for use in the treatment of infections caused by bacteria, in the veterinary field, the composition comprising chlorhexidine or a salt thereof, and at least one peptide, said at least one peptide consisting of 10-50 amino acids, wherein at least two amino acids are basic amino acids selected from Lys, His, Arg, or a combination thereof, and selected from SEQ.ID.No. 1-54, specifically SEQ.ID.No. 1, and wherein said bacteria are *Pseudomonas aeruginosa* and *Staphylococcus aureus.*

In the composition of the invention, chlorhexidine is as a free base or in the form of a salt thereof; "salt of chlorhexidine" means dihydrochloride, diacetate, digluconate or a mixture thereof. Preferably, the composition of the invention comprises up to 0.05 g/ml of chlorhexidine or a salt thereof and up to 12.5 ug/ml of said at least one peptide.

According to a preferred embodiment, the composition of the invention comprises up to 0.03 g/ml of chlorhexidine or a salt thereof and up to 6 ug/ml of said at least one peptide.

Said at least one cationic peptide selected from SEQ.ID.No. 1-54 is a peptide having a sequence A-B-C-D-C'-B'-A', where:
- each unit A independently consists of 1-3 amino acids;
- each unit B independently consists of a sulfur-containing amino acid;
- each unit C independently consists of 5 amino acids selected from both the group (a) of hydrophobic amino acids and the group (b) of basic amino acids or hydrogen bond-forming amino acids;
- unit D consists of glycine and a basic amino acid,
where:
(i) said hydrophobic amino acids are selected from: Ala, Phe, Ile, Leu, Pro, Tyr, Trp and Val;
(ii) said basic amino acids are selected from: Lys, His, Arg;
(iii) said hydrogen bond-forming amino acids are selected from Asn, Gln, Ser, Thr; and where the substructure C-D-C' contains a total of 5 to 9 points of alternation between amino acid of the group (a) and amino acid of the group (b), or vice versa.

It has in fact been surprisingly observed that the combination of chlorhexidine or a salt thereof with said at least one peptide generates an unexpected and very significant synergistic effect, as demonstrated in the Examples below. In particular, it has been shown that it is possible to use extremely low concentrations of chlorhexidine, as well as extremely low concentrations of peptide, to obtain more than satisfactory results, especially considering that at the same low concentrations the single components have shown no activity. The possibility of drastically reducing the concentration of chlorhexidine consequently drastically reduces the risk of triggering mechanisms of resistance by the microorganisms and the onset of side effects, with evident advantages not only from the economic and environmental points of view, but also and especially from the points of view of efficacy and safety of use.

Said at least one peptide has a length of 15 to 21 amino acids, can be cyclized by formation of a disulfide bridge between two sulfur-containing amino acids, suitably located in the proximity of the -NH₂ terminal and -COOH terminal regions, in the cyclized form taking a twisted beta sheet shape. Furthermore, the central portion of the peptide is characterized by the presence of several charged amino acids, in part or totally alternating with neutral amino acids. More specifically, said peptide has a sequence of the A-B-C-D-C'-B'-A' type, where: units A and A' represent the -NH₂ terminal and -COOH terminal regions, respectively; units B and B' consist of sulfur-containing amino acids; units C consist of 5 amino acids selected from: (a) hydrophobic amino acids and (b) basic or hydrogen bond-forming amino acids; unit D consists of a basic amino acid and glycine. Substructure C-D-C' is characterized by containing a total of 5 to 9 points of alternation between amino acid of the group (a) and amino acid of the group (b), or vice versa.

The hairpin conformation of the peptide, due to the group D in the central portion of the sequence, juxtaposes the two sulfur-containing amino acids B, which in a suitable environment (air or oxidizing conditions) form the disulfide bridge. The cyclization of the structure contributes to the stability of the peptide and to the resistance to the action of bacterial peptidases, therefore, preferably said at least one peptide is in a cyclized form by formation of a disulfide bridge between the two units B. The above-mentioned sequence comprises a percentage of hydrophobic amino acids so as not to perturb the membranes of the eukaryotic cells, while ensuring low/zero toxicity for such cells. Furthermore, the arrangement of the hydrophobic amino acids in discrete regions, separated by charged amino acids, i.e. basic and/or hydrogen bond-forming amino acids, imparts greater efficacy to the peptides and possibly also a higher salt-insensitivity. Finally, such peptides show a high solubility in aqueous solvents.

Said at least one peptide is easily synthesized, proteolytically stable, substantially salt-insensitive, non-hemolytic and non-cytotoxic to eukaryotic cells.

The term "peptide" is defined in the present invention as a plurality of amino acid residues linked by peptide bonds. It has the same meaning as polypeptide and protein and can be used interchangeably. The polypeptide-forming amino acids are identified herein without distinction either by their full name or by the relevant official international abbreviation (1 or 3 letter code).

The term "series" is defined as all possible variations of the at least one peptide wherein one or more amino acids of the peptide sequence are substituted with a homologous amino acid so that the properties of the peptides are maintained, though not necessarily at the same level. Another variant may have greater or lesser activity and/or a wider spectrum (for example, an activity against a wider range of microbes) or be more specific to a particular microorganism. Preferably, conservative substitutions of amino acids are carried out in one or more amino acid residues.

The term "beta sheet" refers to the three-dimensional structure of the cyclized peptide, where each strand has a clockwise twist of about 30°; such a geometry is the compromise between the conformational energy optimization of the two strands forming the sheet and the retention of the geometry of the intra-strand hydrogen bonds. In the above sequence, units A, B, C, D, C', B', A' are connected in the order A-B-C-D-C'-B'-A' to form a linear sequence; such a sequence can be cyclized or is cyclized by a direct bond between the two units B. In the above structure, the units marked with the same letter are not necessarily equal to each other but may contain different amino acids; it follows that, compared to the central group D, the invention includes both symmetric and asymmetric peptides.

Units A represent the peptide terminal regions: unit A indicates the -NH₂ terminal region while unit A' indicates the -COOH terminal region. Each unit A and A' independently consists of 1, 2 or 3 amino acids; preferably, moreover, the total number of amino acids in these two units A and A' is equal to 3, or 2+ 1 or 1+2, respectively.

Preferably, unit A comprises at least one lysine and one amino acid selected from glycine, alanine, leucine, isoleucine, valine, tryptophan, histidine and arginine.

Preferably, unit A' comprises at least one amino acid selected from glycine, alanine, leucine, isoleucine, valine, phenylalanine, tyrosine, tryptophan, histidine, arginine and lysine.

Units B denote a sulfur-containing amino acid, in particular cysteine or methionine. Units B are involved in the formation of the disulfide bridge responsible for the cyclization of the peptide. The cyclization may be carried out at the time of the synthesis of the peptide or it may occur later in the presence of an adequate environmental oxygen supply. Preferably, both units B and B' are cysteine. Unit D denotes a basic amino acid and glycine, preferably with: basic amino acid→glycine sequence, in the direction A→A'; the basic amino acid here is preferably arginine. The glycine present in D, usefully supported by arginine, allows the hairpin conformation of the peptide and, in combination with units C linked thereto, an adequate spacing of units B forming the disulfide bridge.

Units C consist, independently of each other, of 5 amino acids selected from:
(a) both the group of hydrophobic amino acids,
(b) and the group of basic or hydrogen bond-forming amino acids.

Hydrophobic amino acids of group (a) are selected from: alanine, phenylalanine, isoleucine, leucine, proline, tyrosine, tryptophan and valine. In relation to the total number of all amino acids of the peptide, they preferably represent between 30 and 50%, more preferably between 35 and 45%, for example between 39 and 43%.

The basic amino acids of group (b) are selected from: lysine, histidine, arginine.

The hydrogen bond-forming amino acids of group (b) are selected from asparagine, glutamine, serine, threonine.

Preferably, each unit A and A' of the peptide independently consists of 1 or 2 amino acids and at least one of the units C and C' comprises Lys.

More preferably, both units C and C' of the peptide comprise Lys.

In a preferred embodiment, each unit C contains both amino acids of group (a) and amino acids of group (b); basic amino acids, hydrogen bond-forming amino acids or both may be freely used as members of group (b). In a preferred variant, units C contain 50-100% of basic amino acids as members of group (b).

An essential feature of units C is the high degree of alternation between the positively charged amino acids of group (b) and the electrically neutral amino acids of group (a). In particular, the sequence C-D-C' contains 5 to 9 points of alternation between: (a) hydrophobic amino acid and (b) basic or hydrogen bond-forming amino acid, or vice versa. The number of "points of alternation" is equal to the number of peptide bonds which, in the sequence C-D-C', separate an amino acid of group (a) from an amino acid of group (b) directly linked thereto: for example, the sequence Ala-His-Ala-Thr-Phe contains 4 points of alternation, corresponding to the 4 peptide bonds present in the sequence; conversely, a sequence Lys-Ala-Phe-Lys-Phe contains only 3 points of alternation: this is because Ala and Phe belong to the same class (a), and therefore the Ala-Phe bond does not count as "point of alternation". For the purposes of the present invention, the "points of alternation" also include the bond between the basic amino acid present in D and the amino acid of unit C linked thereto, if said amino acid is a hydrophobic amino acid; conversely, the bonds involving glycine and the sulfur-containing amino acids do not count as "points of alternation", irrespective of the amino acid linked thereto.

Therefore in units C, amino acids (a) and (b) are typically alternating; however, this does not exclude the possibility of limited adjacencies between amino acids of the same group ((a) or (b)), provided that said number of points of alternation in the sequence C-D-C' is respected.

In a preferred embodiment, unit D of the peptide is -Arg-Gly-.

In a further preferred embodiment, the amino acid adjacent to Gly of unit D of the peptide is a hydrophobic amino acid, preferably an aromatic hydrophobic amino acid.

A preferred subgroup of peptides having the sequence described above is the subgroup containing a total of 17 amino acids, wherein the units A and A' independently consist of 1 or 2 amino acids, the units B and B' are both Cys, at least one of the units C and C' comprises Lys, all the amino acids in position 6, 8, 13 (numbered from A to A') belong to said group (i) of hydrophobic amino acids, and amino acids in position 9 and 10 are Arg and Gly.

A mostly preferred subgroup is characterized by containing, in addition to the characteristics listed above, a hydrophobic amino acid specifically in position 11 (always numbering from A to A'). This characteristic is particularly useful to increase the salt-insensitivity of the peptide, i.e. the retention of its antibacterial action also in the presence of high concentrations of salt. This property is of particular importance since the membranolytic activity of antimicrobial peptides is generally based on the electrostatic interaction with the negatively charged bacterial or fungal membranes; normally, the presence of free ions (e.g. Na⁺, Cl⁻, commonly found in the assay medium or in the body/disease fluids) masks the negative charge present on the bacterial membrane, thus reducing the peptide binding efficiency and therefore the efficacy of treatment. The present peptides are not affected by this undesired phenomenon, thus keeping a significant efficacy (particularly against Gram-negative bacteria) in the presence of high environmental ion concentrations.

All the amino acids present in the at least one peptide may be present without distinction either in form D- or L-; preferably, they are mainly (i.e. more than 50%) or totally in the form L.

All the amino acids may be used in their natural state or in the form of synthetic derivatives thereof. A preferred group of peptides is that in which:
- unit A (-NH₂ terminal) contains 2 amino acids and unit A' (-COOH terminal) contains 1 amino acid, and
- both units B and B' denote cysteine.

Although according to the present invention the at least one peptide is named AMP2041 (SEQ.ID. No.1), further peptides are here disclosed and named AMP72 (SEQ.ID.No.2) and named AMP126 (SEQ.ID.No.3). Further peptides disclosed are those of sequences SEQ.ID. No. 4-54 as described herein, in particular AMP289 (SEQ.ID. No.4), AMP944 (SEQ.ID. No.5), AMP573 (SEQ.ID. No.22), AMP1360 (SEQ.ID. No.8), AMP1189 (SEQ.ID. No.7), AMP1188 (SEQ.ID. No.6), AMP16 (SEQ.ID. No.9), AMP51 (SEQ.ID. No.10).

Disclosed are also the nearby homologues of each of said SEQ.ID. No. 4-54, characterized by being modified in a single amino acid in any position between no. 1 and 17, where said modification does not affect the amino acids in position 3, 9, 10, 16; the change consists in replacing said amino acid with another amino acid selected from the 20 natural amino acids; preferably, the amino acid is substituted with another amino acid belonging to the same category (a) or (b) as defined above: for example, Ala is substituted with Leu; or Ser is substituted with Lys or Thr, etc.

Said at least one peptide is generally a synthetic peptide synthesized *in vitro* by using chemical methods known in the art. For example, it is prepared by using synthesis procedures on solid phase, liquid phase, peptide condensation or any combination of the above techniques. The amino acids, which form said at least one peptide, may be natural or synthetic. The amino acids used for the peptide synthesis may be amino acids wherein the α-amino-terminal is protected by the acid-labile group N-α-t-butyloxycarbonyl (Boc) according to Merrifield's work (J. Am. Chem. Soc., 85: 2149-2154,1963) or by the base-labile 9-fluorenylmethoxycarbonyl (Fmoc) as described by Carpino and Han (J. Org. Chem., 37:3403-3409, 1972). Both Boc- and Fmoc-protected amino acids can be obtained from commercial sources, such as Fluka, Sigma-Aldrich Bachem, Advanced Chemtech, Cambridge Biochemical Research.

In general, the methods of chemical synthesis on solid phase consist, according to M. Bodansky, `Principi di sintesi peptidica' (Springer-Verlag, Berlin 1984) or JM Stewart and JD Young, `Solid Phase Peptide Synthesis' (Pierce Chemical Co., Rockford, Illinois 1984), in the sequential addition of one or more amino acids to the growing peptide chain. Generally, the amino group or carboxyl group of the first amino acid is protected by an optimal protective group. The first protected amino acid is attached to a solid inert support such as a resin. The protective group is then removed from the resin-bonded residue and the subsequent amino acids (suitably protected) are added sequentially. After reaching the number of amino acids, all remaining protective groups (and any solid support) are removed sequentially or simultaneously, to have the final peptide.

More than one amino acid at a time may be added to the growing chain, for example by coupling (in suitable experimental conditions which prevent the formation of racemes, due to the presence of chiral centers) a protected tripeptide with a suitably protected dipeptide to form, after deprotection, a pentapeptide as described, for example, by Merrifield in G. Barany and RB Merrifield, 'I peptidi: Analisi, Sintesi, Biologia', Ed. E. and J. Gross Meienhofer, vol. 2, (Academic Press, New York, 1980, pp 3-254).

Said peptides can be synthesized by companies providing the custom peptide synthesis service, such as, but not limited to, Sigma-Aldrich (St. Louis, MO, USA), SelleckChem (Houston, TX, USA), Invitrogen (Grand Island, NY, USA), Abgent, OX144RY, Oxfordshire (United Kingdom).

The degree of purity of the peptide compound can be determined by various methods, including the identification of HPLC peaks. Preferably, a peptide which produces a single peak of height and width of at least 75% of the incoming material on an HPLC column is preferred. Even more preferred is a peptide which produces a single peak which is at least 87%, at least 90%, at least 99% or even 99.5% of the incoming material on an HPLC column.

To ensure that the peptide obtained by using one of the above synthesis techniques is the desired peptide for the uses or the formulations described hereafter in the present invention, the analysis of the composition of the peptide is carried out with the aid of different analytical methods known in the art. The analysis of the composition can be carried out, for example, by using the high resolution mass spectrometry to determine the molecular weight of the peptide. Alternatively, the amino acid contents in a peptide can be confirmed by hydrolyzing the peptide in acidic solution to identify and quantify the components of the mixture using HPLC, or an amino acid analyzer. Equally useful are thin layer chromatographic methods, which may also be used to identify one or more constituent groups or residues of a desired peptide.

Another preferred aspect of the at least one peptide relates to the polar angle between 90° and 180°, preferably between 91° and 179°, more preferably between 104° and 115°. The term "polar angle" means, in the present document, the measure of the angle formed between the polar and non-polar side of a peptide conformed in an amphiphilic structure.

Another preferred aspect of the at least one peptide relates to the Boman index between -1 and +4. Preferably, between -0.5 and +3 and even more preferably between +1 and +2.5, for example between +1.1 and +2.0. The term "Boman index" is defined in the present invention as the sum of the transfer energies from water to the cyclohexane of the side chains of the single amino acids forming the peptides divided by the total number of residues, according to what described by Radzeka and Wolfenden (1988) in "Comparing the polarities of amino acids: side-chain distribution coefficients between vapor phase, cyclohexane, 1-octanol and neutral aqueous solution." (Biochemistry 27:1664-1670). The calculated values are negative but the sign (+ or-) is reversed.

Another preferred aspect relates to the percentage of solubility in water of the at least one peptide in the range between 40% and 90%, preferably between 91% and 97%, even more preferably between 97.5% and 100%, such as 98%. The estimated percentage of solubility is calculated by using the two-parameter solubility model of Wilkinson-Harris, as described in Wilkinson DL and Harrison RG (1991) Bio/Technology 9, 443-448.

In further embodiments, the composition of the invention comprises chlorhexidine or a salt thereof, at least one peptide selected from human Beta-defensin, LL-37, Lactoferricin B, Lactoferrin (f 17-41), Temporin A, Temporin B, Temporin L, Indolicin, Melittin, Protegrin-1, Protegrin-2, Protegrin-3, Protegrin-4, Protegrin-5, Magainin 2, RTD-1, RTD-2, RTD-3, RTD-4, RTD-5, Arenicin-1, Arenicin-2 Arenicin-3, Dermcidin, Cecropin, Andropin, Moricin, Ceratotoxin, Dermaseptin, Bombinin, preferably Maximin H1, Maximin H2, Maximin H3, Maximin H4 or Maximin H5, Esculentin, Ranalexin, Buforin II, human CAP18, Abaecin, Apidaecin, Profenin, Bactenecin, Brevinin-1, Brevinin-2, Tachyplesin, or Drosomycin, and at least one cationic peptide having a sequence A-B-C-D-C'-B'-A', as described above, i.e. the peptide AMP2041.

In other preferred embodiments, the composition of the invention further comprises a buffer solution comprising a buffer compound selected from TRIS (or tris(hydroxymethyl)aminomethane), PIPES (or piperazin-1,4-bis (2-ethanesulfonate acid)), TRIS*HCl, HEPES (or 4-2-hydroxyethyl-1-piperazinyl-ethanesulfonic acid), sodium phosphate monobasic and dibasic acid, or citric acid, and comprising a sequestering agent selected from EGTA (ethyleneglycoltetraacetic acid), EDTA (ethylenediaminetetraacetic acid) or an anhydrous or hydrated-salt form thereof, calcium disodium EDTA or a hydrated form thereof, diammonium EDTA or a hydrated form thereof, dipotassium EDTA or a hydrated form thereof, disodium EDTA or a hydrated or dihydrated form thereof, TEA-EDTA (EDTA salt of mono (triethanolamine)), tetrasodium EDTA, tripotassium EDTA, trisodium EDTA, HEDTA (hydroxyethyl-ethylenediaminotriacetic acid), HEDTA-EDTA, and mixtures thereof.

Suitable concentrations of buffer compound are up to 1 g/ml and suitable concentrations of sequestering agent are up to 0.5 g/ml.

Preferably, when the at least one peptide is a cationic peptide having a sequence A-B-C-D-C'-B'-A', the composition of the invention comprises up to 0.0025 g/ml of chlorhexidine or a salt thereof, up to 10.0 ug/ml of at least one peptide, up to 0.5 g/ml of buffer compound and up to 0.2 g/ml of sequestering agent. More preferably, the composition of the invention comprises up to 0.002 g/ml of chlorhexidine or a salt thereof, up to 5.0 ug/ml of at least one peptide selected from SEQ.ID. No. 1-54, up to 0.1 g/ml of buffer compound, and up to 0.01 g/ml of sequestering agent.

When said at least one peptide is selected from one of the other peptides described above, preferably the composition of the invention comprises from 0.8 ug/ml to 0.0025 g/ml of chlorhexidine or a salt thereof, up to 10.0 ug/ml of at least one peptide, up to 0.5 g/ml of buffer compound and up to 0.2 g/ml of sequestering agent.

In a particularly preferred embodiment, said buffer solution comprises TRIS and EDTA disodium dihydrate.

As will be seen in the following examples, the further presence of the buffer solution surprisingly allows a further increase in the efficacy and activity of the composition itself.

The invention relates to a composition for use in the treatment of an infection caused by bacteria as defined in the claims. In a first variant, the treatment is directed in particular against the group of gram-negative bacteria; in a second variant, the treatment is directed against the group of gram-positive bacteria. The term "treatment" refers to the effects of the composition of the invention, which is able to impart a benefit to patients suffering from an infectious disease, such as an improvement in the patient's condition or delay in the progression of the disease. In the present document, the term "infection" or its synonym "infectious disease" refers to the invasion, colonization and/or multiplication of a microorganism into or on another host organism. The term "microbial infection" means an infectious disease caused by a pathogen, defined above, e.g. a bacterium, a parasite, a protozoan, a virus or a fungus, including yeasts. In the present document, the term "subject" defines any multicellular organism, including a human being, an animal, an insect or a plant, which may be infected with a microorganism. Preferably, the subject is any animal organism, such as a human being or animal, which may be infected with a microorganism against which an antimicrobial peptide or a variant thereof is active. A pathogenic bacterium, as defined above, can result from one of the bacterial species such as: Staphylococcus spp, e.g. Staphylococcus aureus (e.g. Staphylococcus aureus ATCC 25923), Enterococcus spp, e.g. Enterococcus faecalis ATCC 29212; Pseudomonas spp., e.g. Pseudomonas aeruginosa ATCC 27853; Mycobacterium spp, e.g. Mycobacterium tuberculosis; Enterobacter spp; Campylobacter spp; Salmonella spp (e.g. Salmonella enteritidis ATCC13076); Streptococcus spp, e.g. Streptococcus group A or B, Streptoccocus pneumoniae, Helicobacter spp, e.g. Helicobacter pylori; Neisseria spp, e.g. Neisseria gonorrea, Neisseria meningitidis; Borrelia burgdorferi, Shigella spp, e.g. Shigella flexneri; Escherichia coli (ATCC 25922); Haemophilus spp, e.g. Haemophilus influenzae; Francisellatularensis, Bacillus spp, e.g. Bacillus anthracis; Clostridium spp, Clostridium botulinum, Yersinia spp, e.g. Yersinia pestis; Treponema spp; Burkholderia spp; e.g. Burkholderia cepacia ATCC 17759, B. mallei and B. pseudomallei; Stenotrophomonas spp, e.g. Stenotrophomonas maltophilia ATCC 13637 According to the invention as defined by the claims, only the treatment of a bacterial infection in the veterinary field caused by *Pseudomonas aeruginosa* or *Staphylococcus aureus* is envisaged.

The biological activity of the composition of the invention against microorganisms was determined, for example, on gram-negative bacteria, with reference to bacteria such as Pseudomonas aeruginosa.

In particular, Pseudomonas aeruginosa is a problematic gram-negative bacterium due to its invasiveness and heterogeneous resistance to antibacterial chemotherapy. This microorganism is responsible for severe infections and causes significant morbidity in subjects immunocompromised from viral infections such as HIV, cancer chemotherapy or immunosuppressive therapies. Moreover, this bacterium is often the causative agent of severe infectious diseases of the lower respiratory tract, urinary tract, skin lesions (sores, ulcers) in young people, including subjects suffering from cystic fibrosis, and elderly hospitalized patients. In recent years, the incidence of Pseudomonas infections in cystic fibrosis has been significantly increasing.

A fungal pathogen can be derived from one of the fungi (including yeasts) belonging to the group comprising the geni Candida spp. (e.g. C.albicans), Epidermophyton spp. Exophiala spp. Microsporum spp. Trichophyton spp. (e.g. T.rubrum e T. interdigitale), Tinea spp. Aspergillus spp. Blastomyces spp. Blastoschizomyces spp. Coccidioides spp. Cryptococcus spp. (e.g. Cryptococcus neoformans), Histoplasma spp. Paracoccidiomyces spp. Sporotrix spp. Absidia spp. Cladophialophora spp. Fonsecaea spp. Phialophora spp. Lacazia spp. Arthrographis spp. Acremonium spp. Actinomadura spp. Apophysomyces spp., Emmonsia spp. Basidiobolus spp. Beauveria spp. Chrysosporium spp. Conidiobolus spp. Cunninghamella spp. Fusarium spp. Geotrichum spp. Graphium spp. Leptosphaeria spp. Malassezia spp. (e.g. Malassezia furfur), Mucor spp. Neotestudina spp. Nocardia spp., Nocardiopsis spp. Paecilomyces spp. Phoma spp. Piedraia spp. Pneumocystis spp. Pseudallescheria spp. Pyrenochaeta spp. Rhizomucor spp. Rhizopus spp. Rhodotorula spp. Saccharomyces spp. Scedosporium spp. Scopulariopsis spp. Sporobolomyces spp. Syncephalastrum spp. Trichoderma spp. Trichosporon spp. Ulocladium spp. Ustilago spp. Verticillium spp. Wangiella spp .

The composition of the present invention may also be useful in the treatment of infections usually associated with the skin, such as ulcers and lesions and skin wounds, cuts or burns.

A further preferred aspect of the invention indicates that the composition is useful in the treatment of bacterial skin infections or pyodermite.

Another aspect involves the use of the composition of the invention in the treatment of (clinical or surgical) diseases complicated by bacterial suprainfections, such as infections associated with the mucosa, infections associated with the gastrointestinal, genitourinary tract, infections of the urinary tract (e.g. pyelonephritis, cystitis, urethritis) or respiratory infections, for example cystic fibrosis.

Mammals, birds and, in general, other animals can be treated with the peptides described in the present invention. Mammals and birds include, but are not limited to, humans, dogs, cats and pet birds and productive livestock such as horses, cattle, sheep, goats, pigs, chickens and turkeys and poultry.

Another preferred aspect relates to the treatment of glanders in equidae and melioidosis in carnivores and Pseudomonas aeruginosa infections in pets and productive livestock.

Another aspect relates to the treatment of Bordetella spp infections in pet animals and productive livestock; Moraxella spp infections; Francisella spp infections, Brucella spp infections, Campylobacter spp. infections, Pasteurella spp. infections; Actinobacillus spp. infections (Actinobacillosis); Haemophilus spp. infections; Streptococcus spp. infections (including mastitis in cattle, strangles); Staphylococcus spp. infections (including mastitis, pyoderma, endometritis); Bacillus spp. infections, including anthrax; Clostridium spp infections, including tetanus, botulism and symptomatic anthrax; Listeria spp. infections (listeriosis); Erysipelothrix spp. infections, including erysipelas suis; Leptospira spp. infections, Serpulina (surface necrotic enteritis), Treponema spp. (rabbit syphilis), Borrelia spp. in pet animals and productive livestock.

The composition of the invention may be prepared by procedures described in the art and with excipients known and readily available.

In the present document, the term "excipient" means a compound or an optimal mixture thereof for use in a formulation prepared for the treatment of a specific infectious disease or conditions associated therewith. For example, an excipient for use in a pharmaceutical formulation must not generally cause an adverse response in a subject. The excipient, as described above, should not significantly inhibit the relevant biological activity of the active compound. For example, an excipient does not significantly inhibit the antimicrobial activity of the composition of the present invention or a variant thereof.

Suitable excipients are sweeteners, diluents, disintegrants, glidants, coloring agents, binders, lubricants, stabilizers, adsorbents, preservatives, surfactants, humectants, perfumes, sebo-reducing agents, keratinolitic agents, softeners, restructuring agents, film-forming substances, emulsifiers, wetting agents, release retardants or mixtures thereof.

Preferably, the surfactants are amphoteric surfactants in a concentration not higher than 15% (0.15 g/ml), non-ionic surfactants in a concentration not higher than 15% (0.15 g/ml) or cationic surfactants in a concentration not higher than 15% (0.15 g/ml).

Emulsifiers, humectants, sebo-reducing agents, keratinolitics, softeners, restructuring agents are preferably in a concentration not higher than 15% (0.15 g/ml).

Lubricants and film-forming agents are preferably in a concentration not higher than 3% (0.03 g/ml). Preservatives are preferably in a concentration not higher than 1% (0.01 g/ml).

Coloring agents are preferably in a concentration not higher than 2% (0.02 g/ml).

Perfumes are preferably in a concentration not higher than 5% (0.05 g/ml).

Alternatively or in addition, the excipient can comprise a compound, such as a protease inhibitor, which increases the activity or half-life of the at least one peptide. In another example, the excipient may include or be itself an additional antimicrobial molecule and/or an anti-inflammatory molecule. The composition of the invention may also take the form of an aqueous solution, an anhydrous form or a dispersion, or alternatively the form of an emulsion, a suspension, an ointment, a cream, a paste, a gel or a salve.

The composition of the invention may also take the form of a topical product for cleansing and cleaning of both human beings and animals, such as a gel, a spray, an aqueous solution, a shampoo or an otological or dermatological solution.

Said at least one peptide of the composition of the present invention may be formulated in powder form, obtained by aseptic isolation of a sterile solid or by lyophilization of a solution to be reconstituted in the form of solution with the aid of a suitable carrier prior to use, for example water. The composition may be in solid form, such as tablet, mini-tablet, micro-tablet, granule, micro-granule, pellet, multiparticulate, micronized particulate, or it may be in the form of a solution, cream, ointment, salve, paste, oil, emulsion, gel, vials or drops.

The solid forms, such as tablets, may be coated with standard aqueous or non-aqueous techniques. The amount of active compounds in such therapeutically useful compositions is such as to allow a therapeutically effective dosage to be obtained. The active compounds may also be administered by auricular route, for example liquid drops or spray.

The solid forms may also contain a binder such as tragacanth gum, acacia, corn starch or gelatin; excipients such as dicalcium phosphate; a disintegrating agent such as corn starch, potato starch, alginic acid; and a lubricant such as magnesium stearate.

Controlled-release, slow-release or sustained-release forms may be provided.

A liquid form may contain, in addition to the active compounds, methyl and propyl paraben as preservatives, a dye and a flavoring agent.

Compositions for topical administration include ointments, creams, lotions, solutions, pastes, gels, liposomes, nanoparticles, patches, bandages and dressings. In certain embodiments, the topical formulation comprises at least one cutaneous penetration promoter.

The administration of the compositions is carried out at a dosage sufficient to produce the desired therapeutic effect in the subject.

In some embodiments, the effective amounts for topical formulation will depend on the severity of the disease, disorder or condition, previous therapy, the state of health of the individual and the response to the composition.

The composition of the invention can advantageously be in solid form, as described above, so as to achieve extremely and significantly increased storage times compared to corresponding liquid forms, as well as a convenient reduction in the storage volume.

It is understood that any possible combinations of the preferred aspects of the components of the composition as indicated above are likewise described and therefore preferred.

Below are working examples of the present invention.

### EXAMPLES

Examples not involving the peptide AM2041 and the compound chlorhexidine or a salt thereof are provided for reference or comparison only.

### EXAMPLE 1.

### Peptides

The peptides used in the experimental plan are AMP2041, AMP126 and AMP72. They are synthesized by SelleckChem (Houston, TX, USA) and are characterized by a purity of not less than 90%. The freeze-dried peptides are dissolved in phosphate buffer (PB, 10 mM, 0.8709 g/L K₂HPO₄, 0.6804 g/L KH₂PO₄) at a concentration of 1 mg/ml.

### Preparation of the bacterial suspension

For each strain of reference, three to five morphologically similar colonies are selected from blood agar plates and inoculated into tubes containing 6 ml of Brain Heart Infusion broth (DIFCO, USA). The tubes are vortexed and incubated at 37 °C with stirring at 225 rpm for 3-4 hours, until achieving a required degree of turbidity, greater than or equal to 0.5 of the Mc Farland scale. Thereafter, the bacterial suspension is centrifuged at 1000 rpm for 20 minutes. The resulting pellet of bacteria is resuspended in 10mM PB. The turbidity is adjusted with the same buffer solution and checked by measuring the absorbance of the suspension at the spectrophotometer. The absorbance is considered equal to 0.5 of the Mc Farland scale when the optical density at 600 nm is between 0:08-0: 13. At this value, the bacterial suspension contains approximately 10⁸UFC/ml. Two hundred microliters of the assessed and adjusted bacterial suspension are added to 19.8 ml of 10 mM PB to obtain a final dilution of 1:100. Then, within a thirty minutes, 0:05 ml of this suspension (10⁶UFC/ml) are inoculated into each well of an ELISA plate to obtain a final concentration of bacteria of about 5×10⁵UFC/ml.

(*CSLI. Performance standards for antimicrobial disks and dilution susceptibility test form isolated from animals.; CLSI: Wayne, Pennsylvania, USA, 2008.)*

***Preparation of the plates and evaluation of the Minimum Bactericidal Concentration (MBC).***

Fifty microliters of the peptide 400 µg/ml are added to each well of column 1 of a microtiter plate containing 50 microliters of the buffer solution (PB). Serial dilutions are then carried out from column 1 to column 10 to obtain concentrations of peptide between 200 µg/ml and 0.4 µg/ml. Growth and sterility controls are inserted in column 11 and 12, respectively. 50 µl of work bacterial suspension are added to each well, with the exception of the sterility controls, to obtain a final concentration of the peptide of between 100 µg/ml and 0.2 µg/ml. The plate is incubated for 2 hours at 37 °C in air. Subsequently, 20 µl of each dilution are plated on a suitable solid medium and incubated for 24 h at 37 °C to proceed to the count of CFU. The Minimum Bactericidal Concentration (MBC) is considered as the lowest concentration able to kill at least 99.9% of bacteria. All experiments were repeated in triplicate.

### Time-course of the bactericidal activity of the peptides

1×10⁶ CFU of bacterial suspension in exponential growth are resuspended in 100 µl of 10mM PB and brought into contact with the peptides AMP72, AMP126 and AMP 2041 to values close to the MBC and incubated at 37 °C. Subsequently, 20 µl are seeded at different time intervals (every 5' to 30', then every 10' to 60', then, every 30' to 120') on specific media for the different bacterial species being examined. The growth controls are set up in the PB in the absence of peptide and seeded on solid medium at each time interval for counting the CFU. Finally, after an overnight incubation at 37 °C, the colonies are counted. All experiments were repeated in triplicate.

### Evaluation of the bactericidal activity of new formulations: time-killing assay

The time-killing assay was carried out to evaluate the bactericidal effects of the new formulations, using a modified quantitative test of the European standards EN1040 and EN1276. 100 µl of the bacterial suspension are added to 900 µl of the new formulation and aliquots of 50 µl of the mixture are subjected to assay at 0.5, 1, 2.5, 5, 10, 20, 30, 60, 90 and 120 min., respectively. 450 µl of neutralizing solution are added to each sample and left to act at room temperature for 3 minutes. Threreafter, each sample is serially diluted in PBS (pH 7.4) and 50 µl are plated on LB agar and the plates incubated at 37°C for 24 hours for the count of CFU. The sterility control (100 µl PBS in place of the bacterial suspension) and the control in the absence of the new formulation are set up. The test is also carried out in the presence of 0.3% and 3.0% (w/v) BSA to simulate a paraphysiological environment. All experiments were repeated in triplicate.

### RESULTS

The following table gives the results of the tests carried out. In particular, the activity of the single components, namely chlorhexidine (CLX) and peptide AMP 2041, their combination according to the present invention as well as a preferred combination also comprising TRIS-EDTA, was compared.

These tests clearly show the synergistic effect of the chlorhexidine + peptide combination in terms of minimum concentrations observed against the *Pseudomonas aeruginosa* ATCC 27853 bacterium.

| *Pseudomonas aeruginosa* | CC | T0 | T0.5 | T1 | T3 | T5 | T10 |
|---|---|---|---|---|---|---|---|
| CLX 0.000106% | 600 | 600 | 600 | 600 | 600 | 600 | 600 |
| | | Δ=0% | Δ=0% | Δ=0% | Δ=0% | Δ=0% | Δ=0% |
| AMP 2041 0.5µg/ml | 600 | 600 | 600 | 600 | 600 | 600 | 600 |
| | | Δ=0% | Δ=0% | Δ=0% | Δ=0% | Δ=0% | Δ=0% |
| TRIS-EDTA 0.1% | 600 | 600 | 600 | 600 | 600 | 600 | 600 |
| | | Δ=0% | Δ=0% | Δ=0% | Δ=0% | Δ=0% | Δ=0% |
| **CLX 0.000106% + AMP 2041 0.5µg/ml** | 600 | 600 | **480** | **300** | **180** | **108** | **42** |
| | | Δ=0% | **Δ=-20%** | **Δ=-50%** | **Δ=-70%** | **Δ=-82%** | **Δ=-93%** |
| **CLX 0.000106% + AMP 2041 0.5µg/ml + TRIS-EDTA (0.476%-0.126%)** | 600 | 600 | **300** | **150** | **46** | **14** | **0** |
| | | Δ=0% | **Δ=-50%** | **Δ=-75%** | **Δ=-92.3%** | **Δ=-97.7%** | **Δ=-100%** |

where T0=0min, T0.5=0.5min, T1=1min, T2.5=2.5min, T5=5min, T10=10min

As clear from the above Table, the surprising synergistic effect on *Pseudomonas aeruginosa* ATCC 27853 observed for the `chlorhexidine + peptide' combination is advantageously further increased by the presence of TRIS-EDTA.

The same protocol was also applied to test the antibacterial activity against the *Staphylococcus* ATCC 25923 bacterium. The following table shows the results of the tests carried out. Likewise, the activity of the single components, namely chlorhexidine (CLX) and peptide AMP 2041, and of their combination according to the present invention, was compared.

| *Staphylococcus aureus* | CC | T0 | T0.5 | T1 | T3 | T5 | T10 |
|---|---|---|---|---|---|---|---|
| CLX 0.000106% | 600 | 600 | 600 | 600 | 600 | 600 | 600 |
| | | Δ=0% | Δ=0% | Δ=0% | Δ=0% | Δ=0% | Δ=0% |
| AMP 2041 0.5µg/ml | 600 | 600 | 600 | 600 | 600 | 600 | 600 |
| | | Δ=0% | Δ=0% | Δ=0% | Δ=0% | Δ=0% | Δ=0% |
| **CLX 0.000106% + AMP 2041 0.5µg/ml** | 600 | 600 | 600 | **250** | **100** | **4** | **0** |
| | | Δ=0% | Δ=0% | **Δ=-58%** | **Δ=-83%** | **Δ=-99%** | **Δ=-100%** |

where T0=0min, T0.5=0.5min, T1=1min, T2.5=2.5min, T5=5min, T10=10min

It was therefore surprisingly observed that the combination of the invention, ever since 1 minute after, considerably (50-60%) reduced the CFU of both gram-negative bacteria and of gram-positive bacteria. In particular, it was observed that, ever since only 5 minutes after, the reduction was almost complete (99.33%).

### EXAMPLES 2-17.

Below is the preparation of products made according to the present invention. "%" concentration, in the following examples, means "g/100 ml".

### 2A-2L. Preparation of otological veterinary products

Products (with 8 pH or pH 7-7.5) for cleaning and disinfection of the ear were prepared, having the following composition:

| *Ingredients* | % concentration |
|---|---|
| demineralized water | 89.47995 |
| dihydrated disodium EDTA | 0.1 |
| tris-hydroxymethyl-aminomethane | 0.4 |
| propylene glycol | 10 |
| chlorhexidine digluconate | 0.02 |
| peptide | 0.00005 |

wherein "peptide" is:
- Example 2A: AMP2041
- Example 2B: AMP72
- Example 2C: AMP126
- Example 2D: human Beta-defensin 3 (hBD3)
- Example 2E: Lactoferrin (f 17-41)
- Example 2F: Arenicin-1
- Example 2G: RTD-5
- Example 2H: AMP2041 + human Beta-defensin 3 (hBD3) (1:1)
- Example 2I: AMP72 + Lactoferrin (f 17-41) (1:1)
- Example 2L: AMP289

### 3A-3L. Preparation of liquid veterinary shampoos

Liquid shampoos (with pH 5.5-6.5) were prepared, having the following composition:

| *Ingredients* | % concentration |
|---|---|
| demineralized water | 69.45 |
| dihydrated disodium EDTA | 0.4 |
| tris-hydroxymethyl-aminomethane | 1.6 |
| isodecyl alcohol | 9 |
| aminoxide | 11.25 |
| ethoxylated lanolin | 4 |
| isopropyl alcohol | 2.5 |
| perfumed essence | 0.4 |
| color | 0.001 |
| chlorhexidine digluconate | 0.08 |
| peptide | 0.0002 |

wherein "peptide" is:
- Example 3A: AMP2041
- Example 3B: AMP72
- Example 3C: AMP126
- Example 3D: Lactoferricin B
- Example 3E: Protegrin-1
- Example 3F: Indolicin
- Example 3G: RTD-5
- Example 3H: AMP2041 + Indolicin (2:1)
- Example 3I: AMP126 + Protegrin-1 (1:2)
- Example 3L: AMP944

### 4A-4L. Preparation of liquid veterinary shampoos

Liquid shampoos (with pH 5.5-6.5) were prepared, having the following composition:

| *Ingredients* | % concentration |
|---|---|
| demineralized water | 84.65 |
| dihydrated disodium EDTA | 0.4 |
| tris-hydroxymethyl-aminomethane | 1.6 |
| isodecyl alcohol | 0.55 |
| aminoxide | 8 |
| ethoxylated lanolin | 4 |
| isopropyl alcohol | 0.4 |
| perfumed essence | 0.4 |
| color | 0.001 |
| chlorhexidine digluconate | 0.08 |
| peptide | 0.0002 |

wherein "peptide" is:
- Example 4A: AMP2041
- Example 4B: AMP72
- Example 4C: AMP126
- Example 4D: Lactoferricin B
- Example 4E: Protegrin-1
- Example 4F: Indolicin
- Example 4G: RTD-5
- Example 4H: AMP2041 + Indolicin (2:1)
- Example 4I: AMP126 + Protegrin-1 (1:2)
- Example 4L: AMP944

### 5A-5L. Preparation of liquid veterinary shampoos

Liquid shampoos (with pH 5.5-6.5) were prepared, having the following composition:

| *Ingredients* | % concentration |
|---|---|
| demineralized water | 69.471 |
| dihydrated disodium EDTA | 0.4 |
| tris-hydroxymethyl-aminomethane | 1.6 |
| 30% betaine sol. | 3.33 |
| aminoxide | 20 |

| | |
|---|---|
| ethoxylated lanolin | 4 |
| isopropyl alcohol | 0.799 |
| perfumed essence | 0.4 |
| color | 0.001 |
| chlorhexidine digluconate Staphylococcus aureus ATCC 27300 | 0.08 |
| peptide | 0.0002 |

wherein "peptide" is:
- Example 5A: AMP2041
- Example 5B: AMP72
- Example 5C: AMP126
- Example 5D: Lactoferricin B
- Example 5E: Protegrin-1
- Example 5F: Indolicin
- Example 5G: RTD-5
- Example 5H: AMP2041 + Indolicin (2:1)
- Example 5I: AMP126 + Protegrin-1 (1:2)
- Example 5L: AMP944

### 6A-6L. Preparation of viscous veterinary shampoos

Liquid shampoos (with pH 7-9) were prepared, having the following composition:

| *Ingredients* | % concentration |
|---|---|
| demineralized water | 68.471 |
| cellulose | 1 |
| dihydrated disodium EDTA | 0.4 |
| tris-hydroxymethyl-aminomethane | 1.6 |
| 30% betaine sol. | 3.33 |
| aminoxide | 20 |
| ethoxylated lanolin | 4 |
| isopropyl alcohol | 0.799 |
| perfumed essence | 0.4 |
| color | 0.001 |
| chlorhexidine digluconate | 0.08 |
| peptide | 0.0002 |

wherein "peptide" is:
- Example 6A: AMP2041
- Example 6B: AMP72
- Example 6C: AMP126
- Example 6D: Lactoferricin B
- Example 6E: Protegrin-1
- Example 6F: Indolicin
- Example 6G: RTD-5
- Example 6H: AMP2041 + Indolicin (2:1)
- Example 6I: AMP126 + Protegrin-1 (1:2)
- Example 6L: AMP944

### 7A-7L. Preparation of disinfectant solutions

Disinfectant solutions (with pH 5.5-6.5) were prepared, having the following composition:

| *Ingredients* | % concentration |
|---|---|
| water | 86.4 |
| dihydrated disodium EDTA | 0.1 |
| tris-hydroxymethyl-aminomethane | 0.4 |
| isotridecanol ethoxylate | 0.45 |
| glycerin | 7.5 |
| propylene glycol | 5 |
| chlorhexidine digluconate | 0.02 |
| peptide | 0.00005 |

wherein "peptide" is:
- Example 7A: AMP2041
- Example 7B: AMP72
- Example 7C: AMP126
- Example 7D: Lactoferrin (f 17-41)
- Example 7E: Arenicin-2
- Example 7F: Esculentin
- Example 7G: AMP573
- Example 7H: AMP126 + Lactoferrin (f 17-41) (1:1)
- Example 7I: AMP72 + Arenicin-2 (1:1)
- Example 7L: AMP1360

### 8A-8L. Preparation of disinfectant solutions

Disinfectant solutions (with pH 5.5-6.5) were prepared, having the following composition:

| *Ingredients* | % concentration |
|---|---|
| water | 98.8215 |
| dihydrated disodium EDTA | 0.1 |
| tris-hydroxymethyl-aminomethane | 0.4 |
| isodecyl alcohol | 0.225 |
| aminoxide | 0.281 |
| ethoxylated lanolin | 0.1 |
| isopropyl alcohol | 0.0625 |
| chlorhexidine digluconate | 0.02 |
| glycerin | 2 |
| propylene glycol | 2 |
| peptide | 0.00005 |

wherein "peptide" is:
- Example 8A: AMP2041
- Example 8B: AMP72
- Example 8C: AMP126
- Example 8D: AMP16
- Example 8E: Protegrin-2
- Example 8F: human Beta-defensin 3 (hBD3)
- Example 8G: AMP1189
- Example 8H: AMP2041 + human Beta-defensin 3 (hBD3) (3:1)
- Example 8I: AMP126 + Protegrin-2 (1:3)
- Example 8L: AMP1188

### 9A-9L. Preparation of disinfectant solutions

Disinfectant solutions (with pH 5.5-6.5) were prepared, having the following composition:

| *Ingredients* | % concentration |
|---|---|
| water | 98.1425 |
| dihydrated disodium EDTA | 0.1 |
| tris-hydroxymethyl-aminomethane | 0.4 |
| isodecyl alcohol | 0.45 |
| aminoxide | 0.5625 |
| ethoxylated lanolin | 0.2 |
| isopropyl alcohol | 0.125 |
| chlorhexidine digluconate | 0.02 |
| glycerin | 2 |
| propylene glycol | 2 |
| peptide | 0.00005 |

wherein "peptide" is:
- Example 9A: AMP2041
- Example 9B: AMP72
- Example 9C: AMP126
- Example 9D: human Beta-defensin 3 (hBD3)
- Example 9E: Maximin H1
- Example 9F: Dermaseptin
- Example 9G: RTD-5
- Example 9H: AMP2041 + human Beta-defensin 3 (hBD3) (1:2)
- Example 9I: AMP72 + Maximin H1 (2:1)
- Example 9L: AMP51

### 10A-10L. Preparation of gels

Gels (with pH 6.5-7.5) were prepared, having the following composition:

| *Ingredients* | % concentration |
|---|---|
| demineralized water | 92.37 |
| natrosol | 1.1 |
| dihydrated disodium EDTA | 0.1 |
| tris-hydroxymethyl-aminomethane | 0.4 |
| ethoxylated lanolin | 0.5 |
| glycerin | 2 |
| polyvinylpyrrolidone | 1.5 |
| sorbitol | 1.43 |
| isodecyl alcohol | 0.5 |
| dye | 0.0015 |
| chlorhexidine digluconate | 0.02 |
| peptide | 0.00005 |

wherein "peptide" is:
- Example 10A: AMP2041
- Example 10B: AMP72
- Example 10C: AMP126
- Example 10D: human Beta-defensin 3 (hBD3)
- Example 10E: Lactoferrin (f 17-41)
- Example 10F: Arenicin-3
- Example 10G: RTD-5
- Example 10H: AMP2041 + human Beta-defensin 3 (hBD3) (1:1)
- Example 10I: AMP72 + Lactoferrin (f 17-41) (1:1)
- Example 10L: AMP289

### 11A-11L. Preparation of anti-seborrhoeic shampoos

Liquid shampoos (with pH 4.0-5.5) were prepared, having the following composition:

| *Ingredients* | % concentration |
|---|---|
| demineralized water | 36.23 |
| zinc gluconate | 2 |
| dihydrated disodium EDTA | 0.4 |
| tris-hydroxymethyl-aminomethane | 1.6 |
| aminoxide | 27.5 |
| betaine sol. 30% | 18 |
| Cocamide dea | 3.5 |
| salicylic acid | 2 |
| ethoxylated lanolin | 4 |
| fragrance | 0.4 |
| silicone emulsion | 1 |
| silicone emulsion | 0.2 |
| chlorhexidine digluconate | 0.08 |
| lactic acid (as needed) | about 1 |
| sodium chloride (as needed) | about 1.52 |
| peptide | 0.0002 |

wherein "peptide" is:
- Example 11A: AMP2041
- Example 11B: AMP72
- Example 11C: AMP126
- Example 11D: AMP16
- Example 11E: Protegrin-2
- Example 11F: human Beta-defensin 3 (hBD3)
- Example 11G: AMP1189
- Example 11H: AMP2041 + human Beta-defensin 3 (hBD3) (2:1)
- Example 11I: AMP126 + Protegrin-2 (1:2)
- Example 11L: AMP1188

### 12A-12L. Preparation of anti-seborrhoeic shampoos

Liquid shampoos (with pH 4.0-5.5) were prepared, having the following composition:

| *Ingredients* | % concentration |
|---|---|
| demineralized water | 36.23 |
| zinc gluconate | 2 |
| dihydrated disodium EDTA | 0.4 |
| tris-hydroxymethyl-aminomethane | 1.6 |
| aminoxide | 3 |
| betaine sol. 30% | 2.9 |
| Cocamide dea | 0.9 |
| salicylic acid | 0.6 |
| ethoxylated lanolin | 4 |
| fragrance | 0.4 |
| silicone emulsion | 0.7 |
| silicone emulsion | 0.15 |
| chlorhexidine digluconate | 0.08 |
| lactic acid (as needed) | about 1 |
| sodium chloride (as needed) | about 1.52 |
| peptide | 0.0002 |

wherein "peptide" is:
- Example 12A: AMP2041
- Example 12B: AMP72
- Example 12C: AMP126
- Example 12D: AMP16
- Example 12E: Protegrin-2
- Example 12F: human Beta-defensin 3 (hBD3)
- Example 12G: AMP1189
- Example 12H: AMP2041 + human Beta-defensin 3 (hBD3) (2:1)
- Example 121: AMP126 + Protegrin-2 (1:2)
- Example 12L: AMP1188

### 13A-13L. Preparation of an anti-seborrheic solutions

Disinfectant solutions (with pH 5.5-6.5) were prepared, having the following composition:

| *Ingredients* | % concentration |
|---|---|
| demineralized water | 93.22 |
| zinc gluconate | 0.5 |
| dihydrated disodium EDTA | 0.1 |
| tris-hydroxymethyl-aminomethane | 0.4 |
| urea | 0.2 |
| isotridecanol ethoxylate | 1 |
| Cyclosistem d-panthenol | 0.2 |
| aloe glycolic extract | 0.2 |
| ethoxylated lanolin | 1.5 |
| fragrance | 0.4 |
| glycerin | 1.5 |
| chamomile essence | 0.08 |
| chlorhexidine digluconate | 0.02 |
| omega 6 liposome | 1 |
| peptide | 0.0002 |

wherein "peptide" is:
- Example 13A: AMP2041
- Example 13B: AMP72
- Example 13C: AMP126
- Example 13D: AMP16
- Example 13E: Protegrin-2
- Example 13F: human beta-defensin 3 (hBD3)
- Example 13G: AMP1189
- Example 13H: AMP2041 + human Beta-defensin 3 (hBD3) (2:1)
- Example 131: AMP126 + Protegrin-2 (1:2)
- Example 13L: AMP1188

### 14A-14L. Preparation of disinfectants for surfaces and supports

Products (with pH 8) were prepared, intended for cleaning and disinfection of surfaces and supports of the dairy industry equipment, said products having the following composition:

| *Ingredients* | % concentration |
|---|---|
| demineralized water | 99.22995 |
| dihydrated disodium EDTA | 0.1 |
| tris-hydroxymethyl-aminomethane | 0.4 |
| isotridecanol ethoxylate | 0.25 |
| chlorhexidine digluconate | 0.02 |
| peptide | 0.00005 |

wherein "peptide" is:
- Example 14A: AMP2041
- Example 14B: AMP72
- Example 14C: AMP126
- Example 14D: Temporin A
- Example 14E: Melittin
- Example 14F: Magainin 2
- Example 14G: LL-37
- Example 14H: AMP2041 + LL-37 (1:1)
- Example 141: AMP72 + Melittin (1:1)
- Example 14L: AMP944

### 15A-15L. Preparation of disinfectants for surfaces and supports

Products (with pH 8) were prepared, intended for cleaning and disinfection of surfaces and supports of the dairy industry equipment, said products having the following composition:

| *Ingredients* | % concentration |
|---|---|
| demineralized water | 93.8404 |
| dihydrated disodium EDTA | 0.8 |
| tris-hydroxymethyl-aminomethane | 3.2 |
| isotridecanol ethoxylate | 2 |
| chlorhexidine digluconate | 0.16 |
| peptide | 0.0004 |

wherein "peptide" is:
- Example 15A: AMP2041
- Example 15B: AMP72
- Example 15C: AMP126
- Example 15D: Temporin A
- Example 15E: Melittin
- Example 15F: Magainin 2
- Example 15G: LL-37
- Example 15H: AMP2041 + LL-37 (1:1)
- Example 151: AMP72 + Melittin (1:1)
- Example 15L: AMP944

### 16A-16L. Preparation of a nourishing cleansing veterinary creams

Creams (with pH 4.5-8) were prepared, having the following composition:

| *Ingredients* | % concentration |
|---|---|
| demineralized water | 68.471 |
| dihydrated disodium EDTA | 0.4 |
| tris-hydroxymethyl-aminomethane | 1.6 |
| diethylene glycol stearate | 12 |
| 30% betaine sol. | 3.1 |
| aminoxide | 15 |
| vegetable squalene | 3 |
| propylene glycol | 5 |
| perfumed essence | 0.4 |
| color | 0.001 |
| chlorhexidine digluconate | 0.08 |
| peptide | 0.0002 |

wherein "peptide" is:
- Example 16A: AMP2041
- Example 16B: AMP72
- Example 16C: AMP126
- Example 16D: Lactoferricin B
- Example 16E: Protegrin-1
- Example 16F: Indolicin
- Example 16G: RTD-5
- Example 16H: AMP2041 + Indolicin (2:1)
- Example 16I: AMP126 + Protegrin-1 (1:2)
- Example 16L: AMP944

### 17A-17L. Preparation of anti-seborrhoeic shampoos/creams

Viscous shampoos (with pH 4.0-7.5) were prepared, having the following composition:

| *Ingredients* | % concentration |
|---|---|
| demineralized water | 36.23 |
| zinc gluconate | 2 |
| dihydrated disodium EDTA | 0.4 |
| tris-hydroxymethyl-aminomethane | 1.6 |
| aminoxide | 3 |
| betaine sol. 30% | 2.9 |
| Cocamide dea | 0.9 |
| salicylic acid | 0.6 |
| ethoxylated lanolin | 4 |
| fragrance | 0.4 |
| silicone emulsion | 0.7 |
| silicone emulsion | 0.15 |
| chlorhexidine digluconate | 0.08 |
| lactic acid (as needed) | about 1 |
| diethylene glycol stearate | 12 |
| peptide | 0.0002 |

wherein "peptide" is:
- Example 17A: AMP2041
- Example 17B: AMP72
- Example 17C: AMP126
- Example 17D: AMP16
- Example 17E: Protegrin-2
- Example 17F: human Beta-defensin 3 (hBD3)
- Example 17G: AMP1189
- Example 17H: AMP2041 + human Beta-defensin 3 (hBD3) (2:1)
- Example 17I: AMP126 + Protegrin-2 (1:2)
- Example 17L: AMP1188

The advantages achieved by the composition according to the present invention are apparent from the detailed description and from the Examples above. In particular, said composition allows to take advantage of the surprising synergistic effect resulting from the presence of the at least one peptide, so that it is advantageously possible to benefit from the efficacy of chlorhexidine while minimizing the risk of triggering mechanisms of resistance by the microorganisms concerned. In fact, it has been shown that the compositions of the invention lead to definitely quantitative and satisfactory results of CFU reduction in a very short time and with drastically reduced amounts of chlorhexidine. It is also noted that peptides can trigger mechanisms of resistance by the microorganisms concerned, however the combination with chlorhexidine also allows the use of drastically and advantageously low amounts of peptides.

## Claims

1. A composition for use in the treatment of infections caused by bacteria, in the veterinary field, the composition comprising chlorhexidine or a salt thereof, and at least one peptide, said at least one peptide being a cationic peptide having a sequence A-B-C-D-C'-B'-A', wherein:
- each unit A independently consists of 1-3 amino acids;
- each unit B independently consists of a sulfur-containing amino acid;
- each unit C independently consists of 5 amino acids selected from both the group (a) of hydrophobic amino acids and the group (b) of basic amino acids or hydrogen bond-forming amino acids;
- unit D consists of glycine and a basic amino acid,
wherein:
(i) said hydrophobic amino acids are selected from: Ala, Phe, Ile, Leu, Pro, Tyr, Trp and Val;
(ii) said basic amino acids are selected from: Lys, His, Arg;
(iii) said hydrogen bond-forming amino acids are selected from Asn, Gln, Ser, Thr; and where the substructure C-D-C' contains a total of 5 to 9 points of alternation between an amino acid of group (a) and an amino acid of group (b) or vice versa,
and being SEQ.ID.No.1, and
wherein said bacteria are *Pseudomonas aeruginosa* and *Staphylococcus aureus.*

2. The composition for use of claim 1, wherein said salt of chlorhexidine is dihydrochloride, diacetate, digluconate or a mixture thereof.

3. The composition for use of claim 1 or 2, wherein chlorhexidine or a salt thereof is in a concentration up to 0.05 g/ml, and said at least one peptide is in a concentration up to 12,5 ug/ml.

4. The composition for use of claim 3, wherein chlorhexidine or a salt thereof is in a concentration up to 0.03 g/ml, and said at least one peptide is in a concentration up to 6 ug/ml.

5. The composition for use of any one of claims 1-4, further comprising a buffer solution comprising a buffer compound selected from TRIS (or tris(hydroxymethyl)aminomethane), PIPES (or piperazin-1,4-bis (2-ethanesulfonate acid)), HEPES (or 4-2-hydroxyethyl-1-piperazinyl-ethanesulfonic acid), sodium phosphate monobasic and dibasic acid, or citric acid, and comprising a sequestering agent selected from EGTA (ethyleneglycoltetraacetic acid), EDTA (ethylenediaminetetraacetic acid) or an anhydrous or hydrated-salt form thereof, calcium disodium EDTA or a hydrated form thereof, diammonium EDTA or a hydrated form thereof, dipotassium EDTA or a hydrated form thereof, disodium EDTA or a hydrated or dihydrated form thereof, TEA-EDTA (EDTA salt of mono (triethanolamine))tetrasodium EDTA, tripotassium EDTA, trisodium EDTA, HEDTA (hydroxyethyl-ethylenediaminotriacetic acid), HEDTA-EDTA, and mixtures thereof.

6. The composition for use of claim 5, wherein said buffer compound is in a concentration up to 1 g/ml and said sequestering agent is in a concentration up to 0.5 g/ml.

7. The composition for use of claim 6, comprising up to 0.0025 g/ml of chlorhexidine or a salt thereof, up to 10.0 ug/ml of at least one peptide, up to 0.5 g/ml of buffer compound, and up to 0.2 g/ml of sequestering agent.

8. The composition for use of claim 7, comprising up to 0.002 g/ml of chlorhexidine or a salt thereof, up to 5.0 ug/ml of at least one peptide, up to 0.1 g/ml of buffer compound, and up to 0.01 g/ml of sequestering agent.

9. The composition for use of any one of claims 5-8, wherein said buffer solution comprises TRIS and EDTA disodium dihydrate.

10. The composition for use of any one of claims 1-9, further comprising excipients, which are sweeteners, diluents, disintegrants, glidants, coloring agents, binders, lubricants, stabilizers, adsorbents, preservatives, surfactants, humectants, perfumes, sebo-reducing agents, keratinolitic agents, softeners, restructuring agents, film-forming substances, emulsifiers, wetting agents, release retardants or mixtures thereof.

11. The composition for use of any one of claims 1-10, in the form of aqueous solution, anhydrous solution, dispersion, emulsion, suspension, liniment, cream, paste, gel, ointment, shampoo, powder, aerosol, soft or hard capsule, tablet, mini-tablet, micro-tablet, granule, micro-granule, pellet, multiparticulate, micronized particles, pill, syrup, oil, lotion, drops, eye drops, liposomes, nanoparticles, patches, bandages and dressings.

12. The composition for use of claim 11, in a topically administrable form, selected from a gel, a spray, an aqueous solution, a shampoo, and an otological or dermatological solution.

## Patentansprüche

1. Zusammensetzung zur Verwendung bei der Behandlung von durch Bakterien verursachten Infektionen im Veterinärbereich, wobei die Zusammensetzung Chlorhexidin oder ein Salz davon und mindestens ein Peptid umfasst, wobei das mindestens eine Peptid ein kationisches Peptid mit einer Sequenz A-B-C-D-C'-B'-A' ist, wobei:
- jede Einheit A unabhängig aus 1-3 Aminosäuren besteht;
- jede Einheit B unabhängig aus einer schwefelhaltigen Aminosäure besteht;
- jede Einheit C unabhängig aus 5 Aminosäuren besteht, die sowohl aus der Gruppe (a) von hydrophoben Aminosäuren als auch der Gruppe (b) von basischen Aminosäuren oder wasserstoffbrückenbildenden Aminosäuren ausgewählt sind;
- Einheit D aus Glycin und einer basischen Aminosäure besteht,
wobei:
(i) die hydrophoben Aminosäuren aus Folgenden ausgewählt sind: Ala, Phe, Ile, Leu, Pro, Tyr, Trp und Val;
(ii) die basischen Aminosäuren aus Folgenden ausgewählt sind: Lys, His, Arg;
(iii) die wasserstoffbrückenbildenden Aminosäuren aus Asn, Gln, Ser, Thr ausgewählt sind;
und wobei die Unterstruktur C-D-C' insgesamt 5 bis 9 Wechselpunkte zwischen einer Aminosäure der Gruppe (a) und einer Aminosäure der Gruppe (b) oder umgekehrt enthält,
und SEQ ID NO 1 ist, und
wobei die Bakterien *Pseudomonas aeruginosa* und *Staphylococcus aureus* sind.

2. Zusammensetzung zur Verwendung nach Anspruch 1, wobei das Salz von Chlorhexidin Dihydrochlorid, Diacetat, Digluconat oder eine Mischung davon ist.

3. Zusammensetzung zur Verwendung nach Anspruch 1 oder 2, wobei Chlorhexidin oder ein Salz davon in einer Konzentration von bis zu 0,05 g/ml vorliegt und das mindestens eine Peptid in einer Konzentration von bis zu 12,5 ug/ml vorliegt.

4. Zusammensetzung zur Verwendung nach Anspruch 3, wobei Chlorhexidin oder ein Salz davon in einer Konzentration von bis zu 0,03 g/ml vorliegt und das mindestens eine Peptid in einer Konzentration von bis zu 6 ug/ml vorliegt.

5. Zusammensetzung zur Verwendung nach einem der Ansprüche 1-4, ferner umfassend eine Pufferlösung, die eine Pufferverbindung umfasst, die aus TRIS (oder Tris(hydroxymethyl)aminomethan), PIPES (oder Piperazin-1,4-bis(2-ethansulfonatsäure)), HEPES (oder 4-2-Hydroxyethyl-1-piperazinyl-ethansulfonsäure), ein- und zweibasiger Natriumphosphatsäure oder Zitronensäure ausgewählt ist, und ein Sequestriermittel umfasst, das aus EGTA (Ethylenglykoltetraessigsäure), EDTA (Ethylendiamintetraessigsäure) oder einer wasserfreien oder hydratisierten Salzform davon, Calciumdinatrium-EDTA oder einer hydratisierten Form davon, Diammonium-EDTA oder einer hydratisierten Form davon, Dikalium-EDTA oder einer hydratisierten Form davon, Dinatrium-EDTA oder einer hydratisierten oder dihydratisierten Form davon, TEA-EDTA (EDTA-Salz von Mono(triethanolamin))tetranatrium-EDTA, Trikalium-EDTA, Trinatrium-EDTA, HEDTA (Hydroxyethylethylendiaminotriessigsäure), HEDTA-EDTA und Mischungen davon ausgewählt ist.

6. Zusammensetzung zur Verwendung nach Anspruch 5, wobei die Pufferverbindung in einer Konzentration von bis zu 1 g/ml und das Sequestriermittel in einer Konzentration von bis zu 0,5 g/ml vorliegt.

7. Zusammensetzung zur Verwendung nach Anspruch 6, umfassend bis zu 0,0025 g/ml Chlorhexidin oder ein Salz davon, bis zu 10,0 ug/ml mindestens eines Peptids, bis zu 0,5 g/ml Pufferverbindung und bis zu 0,2 g/ml Sequestriermittel.

8. Zusammensetzung zur Verwendung nach Anspruch 7, umfassend bis zu 0,002 g/ml Chlorhexidin oder ein Salz davon, bis zu 5,0 ug/ml mindestens eines Peptids, bis zu 0,1 g/ml Pufferverbindung und bis zu 0,01 g/ml Sequestriermittel.

9. Zusammensetzung zur Verwendung nach einem der Ansprüche 5-8, wobei die Pufferlösung TRIS und EDTA-Dinatriumdihydrat umfasst.

10. Zusammensetzung zur Verwendung nach einem der Ansprüche 1-9, ferner Hilfsstoffe umfassend, die Süßungsmittel, Verdünnungsmittel, Sprengmittel, Fließregulierungsmittel, Farbstoffe, Bindemittel, Schmiermittel, Stabilisatoren, Adsorptionsmittel, Konservierungsmittel, Tenside, Feuchthaltemittel, Parfüme, talgreduzierende Mittel, keratinolytische Mittel, Weichmacher, Restrukturierungsmittel, filmbildende Substanzen, Emulgatoren, Benetzungsmittel, Trennmittel oder Mischungen davon sind.

11. Zusammensetzung zur Verwendung nach einem der Ansprüche 1-10 in der Form einer wässrigen Lösung, wasserfreien Lösung, Dispersion, Emulsion, Suspension, eines Einreibemittels, einer Creme, Paste, eines Gels, einer Salbe, eines Shampoos, Pulvers, Aerosols, einer Weich- oder Hartkapsel, Tablette, Minitablette, Mikrotablette, eines Granulats, Mikrogranulats, von Pellets, Multipartikeln, mikronisierten Partikeln, einer Pille, eines Sirups, eines Öls, einer Lotion, von Tropfen, Augentropfen, Liposomen, Nanopartikeln, Pflastern, Binden und Verbänden.

12. Zusammensetzung zur Verwendung nach Anspruch 11 in einer topisch verabreichbaren Form, die aus einem Gel, einem Spray, einer wässrigen Lösung, einem Shampoo und einer otologischen oder dermatologischen Lösung ausgewählt ist.

## Revendications

1. Composition destinée à être utilisée dans le traitement d'infections provoquées par des bactéries, dans le domaine vétérinaire, la composition comprenant de la chlorhexidine ou un sel de celle-ci, et au moins un peptide, ledit au moins un peptide étant un peptide cationique présentant une séquence A-B-C-D-C'-B'-A', dans laquelle :
- chaque motif A est constitué indépendamment de 1 à 3 acides aminés ;
- chaque motif B est constitué indépendamment d'un acide aminé contenant du soufre ;
- chaque motif C est constitué indépendamment de 5 acides aminés choisis dans à la fois (a) des acides aminés hydrophobes et le groupe (b) des acides aminés basiques ou des acides aminés formant des liaisons hydrogène ;
- le motif D est constitué de glycine et d'un acide aminé basique :
(i) lesdits acides aminés hydrophobes étant choisis parmi : Ala, Phe, Ile, Leu, Pro, Tyr, Trp et Val ;
(ii) lesdits acides aminés basiques étant choisis parmi : Lys, His, Arg ;
(iii) lesdits acides aminés formant des liaisons hydrogène étant choisis parmi Asn, Gln, Ser, Thr ; et
où la structure C-D-C' contient un total de 5 à 9 points d'alternance entre un acide aminé du groupe (a) et un acide aminé du groupe (b) ou vice versa, et étant SEQ ID n° 1, et
lesdites bactéries étant *Pseudomonas aeruginosa* et *Staphylococcus aureus.*

2. Composition destinée à être utilisée selon la revendication 1, ledit sel de chlorhexidine étant le dihydrochlorure, le diacétate, le digluconate ou un mélange de ceux-ci.

3. Composition destinée à être utilisée selon la revendication 1 ou 2, ladite chlorhexidine ou un sel de celle-ci étant à une concentration allant jusqu'à 0,05 g/ml, et ledit au moins un peptide étant à une concentration allant jusqu'à 12,5 µg/ml.

4. Composition destinée à être utilisée selon la revendication 3, ladite chlorhexidine ou un sel de celle-ci étant à une concentration allant jusqu'à 0,03 g/ml, et ledit au moins un peptide étant à une concentration allant jusqu'à 6 µg/ml.

5. Composition destinée à être utilisée selon l'une quelconque des revendications 1 à 4, comprenant en outre une solution tampon comprenant un composé tampon choisi parmi le TRIS (ou tris(hydroxyméthyl)aminométhane), le PIPES (ou pipérazin-1,4-bis (acide 2-éthanesulfonate)), l'HEPES (ou acide 4-2-hydroxyéthyl-1-pipérazinyl-éthanesulfonique), le phosphate de sodium monobasique et un acide dibasique, ou l'acide citrique comprenant un agent séquestrant choisi parmi l'EGTA (acide éthylèneglycoltétraacétique), l'EDTA (acide éthylènediaminetétraacétique) ou une forme de sel anhydre ou hydraté de ceux-ci, l'EDTA de calcium disodique ou une forme hydratée de celui-ci, l'EDTA de diammonium ou une forme hydratée de celui-ci, l'EDTA de dipotassium ou une forme hydratée de celui-ci, l'EDTA de disodium ou une forme hydratée de celui-ci, le TEA-EDTA (sel d'EDTA de mono(triéthanolamine))l'EDTA de tétrasodium, l'EDTA de tripotassium, l'EDTA de trisodium, l'HEDTA (acide hydroxyéthyl-éthylènediaminotriacétique), l'HEDTA-EDTA, et leurs mélanges.

6. Composition destinée à être utilisée selon la revendication 5, ledit composé tampon étant à une concentration allant jusqu' à 1 g/ml et ledit agent séquestrant étant à une concentration allant jusqu'à 0,5 g/ml.

7. Composition destinée à être utilisée selon la revendication 6, comprenant jusqu'à 0,0025 g/ml de chlorhexidine ou d'un sel de celle-ci, jusqu'à 10,0 µg/ml d'au moins un peptide, jusqu'à 0,5 g/ml de composé tampon et jusqu'à 0,2 g/ml d'agent séquestrant.

8. Composition destinée à être utilisée selon la revendication 7, comprenant jusqu'à 0,002 g/ml de chlorhexidine ou d'un sel de celle-ci, jusqu'à 5,0 µg/ml d'au moins un peptide, jusqu'à 0,1 g/ml de composé tampon et jusqu'à 0,01 g/ml d'agent séquestrant.

9. Composition destinée à être utilisée selon l'une quelconque des revendications 5 à 8, ladite solution tampon comprenant du TRIS et de l'EDTA de disodium dihydraté.

10. Composition destinée à être utilisée selon l'une quelconque des revendications 1 à 9, comprenant en outre des excipients, qui sont des édulcorants, des diluants, des délitants, des agents de glissement, des agents colorants, des liants, des lubrifiants, des stabilisants, des adsorbants, des conservateurs, des tensioactifs, des humectants, des parfums, des agents réducteurs de sébum, des agents kératolytiques, des agents adoucissants, des agents restructurants, des substances filmogènes, des émulsifiants, des agents mouillants, des retardateurs de libération ou des mélanges de ceux-ci.

11. Composition destinée à être utilisée selon l'une quelconque des revendications 1 à 10, sous la forme d'une solution aqueuse, d'une solution anhydre, d'une dispersion, d'une émulsion, d'une suspension, d'une pommade, d'une crème, d'une pâte, d'un gel, d'un onguent, d'un shampoing, d'une poudre, d'un aérosol, d'une capsule souple ou dure, d'un comprimé, d'un mini-comprimé, d'un micro-comprimé, d'un granulé, d'un micro-granulé, d'une pastille, d'un composé multiparticulaire, de particules micronisées, d'une pilule, d'un sirop, d'une huile, d'une lotion, de gouttes, des gouttes pour les yeux, de liposomes, de nanoparticules, de timbres dermiques, de bandages et de pansements.

12. Composition destinée à être utilisée selon la revendication 11, sous une forme administrable par voie topique, choisie parmi un gel, un spray, une solution aqueuse, un shampoing, et une solution otologique ou dermatologique.
